# FASCICULE DE BREVET EUROPEEN

(11) **EP 0 915 806 B1**
(45) Date de publication et mention de la délivrance du brevet: **30.05.2001**
(21) Numéro de dépôt: 97931862.3
(22) Date de dépôt: 03.07.1997
(51) Int. Cl.: C01B 15/037, C11D 3/00, A61L 2/00

(54) **SOLUTION STABILISEE ET TAMPONNEE DE PEROXYDE D'HYDROGENE, SON PROCEDE DE FABRICATION ET SON UTILISATION A LA DECONTAMINATION DE LENTILLES DE CONTACT**
STABILISIERTE UND GEPUFFERTE WASSERSTOFFPEROXIDLÖSUNG, VERFAHREN ZUR DEREN HERSTELLUNG UND VERWENDUNG ZUR DEKONTAMINATION VON KONTAKTLINSEN
STABILISED AND BUFFERED HYDROGEN PEROXIDE SOLUTION, METHOD FOR MAKING SAME AND USE THEREOF FOR DECONTAMINATING CONTACT LENSES

(30) Priorité: 29.07.1996 FR 9609527
(43) Date de publication de la demande: 19.05.1999
(73) Titulaire: ESSILOR INTERNATIONAL (COMPAGNIE GENERALE D'OPTIQUE), F-94227 Charenton cédex (FR)
(72) Inventeur: SOYER, Patrice, F-77390 Guignes (FR); POCHET, Arila, F-77220 Gretz (FR)
(74) Mandataire: Catherine, Alain
(86) Numéro de dépôt international: FR9701195
(87) Numéro de publication internationale: WO9804496

(56) Documents cités:
- EP-A- 0 265 381
- EP-A- 0 354 186
- WO-A-93/13012
- US-A- 4 304 762

## Description

L'invention concerne de manière générale des solutions de désinfection utilisées principalement pour la décontamination de lentilles de contact, en particulier des lentilles de contact hydrophiles et les procédés de décontamination employant de telles solutions.

Plus particulièrement, la présente invention concerne une solution tamponnée et stabilisée de peroxyde d'hydrogène, son procédé d'obtention et son application à la décontamination de lentilles de contact.

Le peroxyde d'hydrogène est un agent oxydant très actif possédant également des propriétés décontaminantes. On a utilisé depuis longtemps des solutions de peroxyde d'hydrogène dans divers buts, incluant le blanchiment, la désinfection, et le nettoyage d'une grande variété de surfaces telle que la peau, les cheveux, les muqueuses et les lentilles de contact, jusqu'à y compris des surfaces et instruments ménagers et industriels.

Il est connu d'utiliser des solutions aqueuses au peroxyde d'hydrogène à 3% pour décontaminer les lentilles de contact. Un exemple d'une telle solution de décontamination est décrit dans le brevet des Etats-Unis n° 3 912 451. Un système exploitant la technique décrite dans ce brevet est d'ailleurs commercialisé sous la marque AOSEPT® .

Toutefois, les solutions de peroxyde d'hydrogène sont éminemment instables, le peroxyde se décomposant rapidement sous l'action de métaux de transition (cuivre, fer, cobalt, manganèse) présents à l'état de trace dans les solutions. Ce phénomène est d'autant plus marqué que le pH de la solution de peroxyde est plus élevé, les solutions de peroxyde d'hydrogène étant naturellement acides.

Il est connu de stabiliser les solutions de peroxyde d'hydrogène en y ajoutant des agents séquestrants qui vont complexer les ions métalliques. On inhibe ainsi la réaction d'oxydo-réduction du peroxyde d'hydrogène.

Des agents séquestrants particulièrement utilisés pour stabiliser les solutions de peroxyde d'hydrogène sont des composés du type comportant des fonctions phosphoniques, sous leur forme acide ou sous leur forme de sel.

Ainsi, le brevet des Etats-Unis n° 3 234 140 décrit l'utilisation d'acides ou de sels amino(triphosphoniques) tels que l'acide aminotris(méthylène phosphonique) pour stabiliser des solutions aqueuses de peroxyde d'hydrogène utilisées pour blanchir des textiles à base de cellulose.

Le comportement des acides phosphoniques ou de leurs sels, dans leurs propriétés stabilisantes du peroxyde d'hydrogène, est très variable et dépend notamment de leur aptitude à la séquestration liée à leur structure chimique.

Ainsi, le brevet des Etats-Unis n° 4 304 672 enseigne que le produit vendu sous la dénomination DEQUEST® 2060 par la société MONSANTO, qui est une solution aqueuse à 50% d'acide d'éthylène triamine penta(méthylène phosphonique) est le stabilisant le plus performant. Ce composé est connu pour ses excellentes propriétés séquestrantes. Ce brevet enseigne également que le produit commercialisé sous la dénomination DEQUEST® 2041, qui est une solution aqueuse d'acide éthylène diamine tétra(méthylène phosphonique), possède aussi d'excellentes propriétés séquestrantes, mais est néanmoins moins actif vis-à-vis de la stabilisation du peroxyde d'hydrogène. D'autre part les produits commercialisés sous la dénomination DEQUEST® 2010 et 2006, respectivement une solution aqueuse à 60% d'acide 2-hydroxy éthylène-1,1-diphosphonique et une solution aqueuse à 29% d'acide amino-tris-méthylène phosphonique et 40% de sel pentasodique de cet acide, qui ont des propriétés complexantes plus faibles, sont peu actifs pour la stabilisation du peroxyde d'hydrogène en milieu basique.

Il existe donc de grandes différences de comportement des composés phosphoniques vis-à-vis de la stabilisation du peroxyde d'hydrogène, certains de ces composés ayant un pouvoir stabilisant pratiquement nul.

Le brevet EP-A-265 381 décrit des solutions de décontamination de lentilles de contact à base de peroxyde d'hydrogène tamponnées à un pH compris entre 5,5 et 7,5 et stabilisées par addition de l'acide d'éthylène triamino penta(méthylène phosphonique) ou ses sels. Préférentiellement, les solutions de décontamination renferment ce seul composé comme unique agent stabilisant.

Le brevet EP-A-289 463 décrit l'utilisation comme agent stabilisant de solution de peroxyde d'hydrogène de la combinaison d'un acide diphosphonique d'alcanol (par exemple, le produit commercialisé sous la dénomination DEQUEST® 2010) et d'un second agent stabilisant choisi parmi la glycérine, un alcool polyvinylique soluble dans l'eau, le propylèneglycol, l'acide polyacrylique, le diéthylèneglycol et le sodium hexamétaphosphate sodium polyphosphate. En l'absence du second agent stabilisant, le composé phosphonique ne permet pas, aux concentrations utilisées, d'obtenir une stabilité à chaud supérieure à 95%.

La demanderesse a découvert qu'en ajoutant aux solutions aqueuses de peroxyde d'hydrogène une combinaison d'un agent séquestrant du type phosphonique essentiellement sous sa forme acide et d'un tampon de borate on obtenait une excellente stabilisation de ces solutions.

En outre, lorsque les solutions selon l'invention sont utilisées dans un procédé de décontamination de lentilles de contact, tel que le procédé décrit dans le brevet des Etats-Unis n° 3 912 451, Ils conduisent, en fin de neutralisation, à des solutions dont le confort est considérablement amélioré par rapport aux solutions de l'art antérieur, les solutions neutralisées ayant une quantité résiduelle de peroxyde d'hydrogène notablement inférieure aux systèmes existants et dont le pH est proche de celui des larmes.

La présente invention a donc pour objet de fournir une solution aqueuse de peroxyde d'hydrogène ayant une stabilité améliorée.

La présente invention a également pour objet l'utilisation d'une solution de peroxyde d'hydrogène telle que définie ci-dessus comme solution de décontamination de lentilles de contact qui, après neutralisation, conduit à une solution neutralisée comportant une quantité moindre de peroxyde d'hydrogène résiduel et ayant un pH proche de celui des larmes, préférentiellement de 7 à 7,5.

La présente invention a enfin pour objet un procédé d'obtention d'une solution aqueuse de peroxyde d'hydrogène stabilisée et tamponnée ayant les propriétés améliorées mentionnées précédemment.

La solution de l'invention est une solution aqueuse, stabilisée et tamponnée, de décontamination des lentilles de contact, comprenant 0,5 à 6% en poids, de préférence 2 à 6% et mieux environ 3% en poids de peroxyde d'hydrogène par rapport au poids total de la composition, caractérisée en ce qu'elle comprend en outre :
a) une quantité stabilisante d'un agent stabilisant à fonctions phosphoniques choisi parmi les composés phosphoniques sous forme acide, les mélanges de composés phosphoniques sous forme acide et les mélanges d'un ou plusieurs composés phosphoniques sous forme acide avec un ou plusieurs de leurs sels à la condition que la teneur en composés phosphoniques sous forme acide dans le mélange soit au moins égale à 90% en poids par rapport au poids total de l'agent stabilisant à fonctions phosphoniques ; et
b) une quantité effective d'un tampon borate, ladite solution ayant un pH compris entre 5 et 6,5.

Dans la présente description et les revendications annexées, l'expression « agent stabilisant à fonctions phosphoniques sous forme acide » désigne, soit un agent stabilisant à fonctions phosphoniques se trouvant totalement sous forme acide, soit un mélange d'agents stabilisants à fonctions phosphoniques sous forme d'acide et de sel à la condition que la quantité d'agent stabilisant sous forme acide soit au moins égale à 90% en poids par rapport au poids total d'agent stabilisant à fonctions phosphoniques.

On entend par « quantité effective d'agent stabilisant à fonctions phosphoniques" une quantité suffisante pour obtenir une stabilisation de la solution finale.

On entend également par "quantité effective de tampon borate" une quantité suffisante de ce tampon pour obtenir un solution finale tamponnée.

De préférence, l'agent stabilisant à fonctions phosphoniques est introduit lors de la préparation des solutions selon l'invention sous forme acide.

Les acides phosphoniques et sels d'acides phosphoniques utilisables dans la présente invention sont des composés connus. Parmi ces acides phosphoniques et sels d'acides phosphoniques on peut citer l'acide amino-tris-méthylène phosphonique, l'acide-1-hydroxyéthylène-1,1-diphosphonique, l'acide éthylènediamine tétra(méthylène phosphonique), l'acide hexaméthylène diamine tétra(méthylène phosphonique), l'acide diéthylène triamine penta(méthylène phosphonique) et les sels des métaux alcalins de ces acides, de préférence leurs sels de sodium. Comme indiqué précédemment, lorsque l'agent stabilisant à fonctions phosphoniques est un mélange de composés phosphoniques sous forme d'acides et de sels d'acides phosphoniques il est important que le mélange comporte au moins 90% en poids de composés phosphoniques sous forme acide par rapport au poids total de l'agent stabilisant à fonctions phosphoniques. De préférence encore, l'agent stabilisant à fonctions phosphoniques comprend uniquement un ou plusieurs composés phosphoniques sous forme acide.

Des acides phosphoniques recommandés dans la présente invention répondent aux formules : 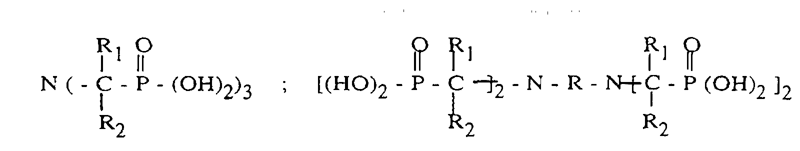 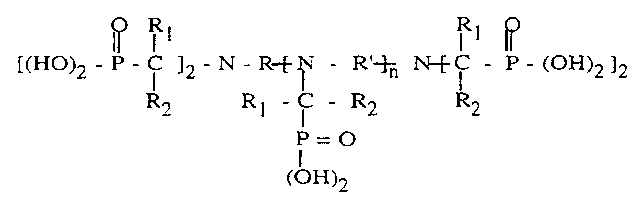 et 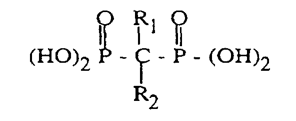 où R₁ et R₂ sont indépendamment l'un de l'autre, l'hydrogène, un groupe alkyle en C₁-C₄ ou un groupe hydroxyle, R et R' sont indépendamment l'un de l'autre un groupe alkylidène en C₁-C₆, et n est un entier de 1 à 4.

Les solutions selon l'invention contiennent en général de 0,003 à 0,2% en poids d'agent stabilisant à fonctions phosphoniques, de préférence 0,006 à 0,1% èt mieux de 0,01 à 0,06% en poids, par rapport au poids total de la composition.

Un autre ingrédient essentiel des solutions conformes à l'invention est le tampon borate. Les tampons borate sont des tampons connus dans la technique. Parmi les composés de base utilisables dans le tampon borate on peut citer l'acide borique, le borate de sodium, le tétraborate de potassium, le métaborate de potassium, le borax Na₂ B₄O₇, 10H₂O et leurs mélanges.

Généralement, la solution selon l'invention comprend 0,05 à 2,5%, de préférence 0,1 à 1,5%, en poids de tampon borate par rapport au poids total de la solution.

Si nécessaire, le pH de la solution est ajusté par addition de quantités appropriées d'un acide ou d'une base, de telle sorte que le pH final de la solution soit compris entre 5 et 6,5, mieux entre 5,5 et 6,5, de préférence encore entre 5,8 et 6,2, le pH optimal étant de 6.

Les solutions de l'invention peuvent en outre comprendre des agents stabilisants classiques, différents des composés phosphoniques ci-dessus. Parmi les agents stabilisants classiques on peut citer l'acide phosphorique, les stannates solubles dans l'eau tels que les stannates de métaux alcalins et d'ammonium, seuls ou en combinaison avec un phosphate, polyphosphate ou métaphosphate soluble dans l'eau, par exemple un sel de métal alcalin ou d'ammonium de ceux-ci, un agent de chélation du type acide amino polycarboxylique, par exemple l'acide éthylène diamine tétraacétique, l'acide nitrilo triacétique ou un sel de ceux-ci soluble dans l'eau, par exemple un sel de métal alcalin ou d'ammonium, ou des mélanges de ceux-ci. Parmi ces agents stabilisants classiques on recommande l'acide phosphorique, les stannates et leurs mélanges. Généralement la solution stabilisée et tamponnée selon l'invention contient une quantité physiologiquement tolérable, par exemple 0,002 à 0,1% en poids par rapport au poids total de la composition, de ces agents stabilisants classiques supplémentaires, lorsqu'ils sont utilisés.

La solution selon l'invention peut également comporter un ou plusieurs agents d'amélioration de la tonicité, physiologiquement acceptable et substantiellement inertes.

De tels agents de l'amélioration de la tonicité comprennent les halogénures des métaux alcalins, les phosphates et les hydrogèno phosphates. Les agents recommandés sont le chlorure de sodium, le phosphate de sodium monobasique et le phosphate de sodium dibasique. Ces agents d'amélioration de la tonicité ont pour fonction d'accroître le niveau de confort de la solution, qui adhère aux lentilles de contact, dans l'oeil du patient, après décomposition du peroxyde d'hydrogène pendant ou après la décontamination des lentilles de contact. Généralement la solution contient une quantité suffisante de ces agents d'amélioration de la tonicité pour que lors de la décomposition de peroxyde d'hydrogène qui y est contenue, la solution résultante soit isotonique, c'est-à-dire équivalente à une solution de chlorure de sodium à 0,9% en poids.

Un autre ingrédient facultatif des solutions selon l'invention est un agent épaississant ou de réglage de la viscosité. On peut utiliser toute substance connue de cette catégorie qui soit physiologiquement acceptable pour l'environnement oculaire. Parmi les agents épaississants classiques, on peut citer l'alcool polyvinylique, l'hydroxyéthyl cellulose, etc. Ces agents épaississants sont présents en quantités suffisantes pour élever la viscosité de la solution finale jusqu'à environ 1000 cps, de préférence jusqu'à 100 cps.

La solution de l'invention peut en outre inclure un agent filmogène. De tels agents filmogènes sont bien connus et sont par exemple des polymères et copolymères cationiques.

Le cas échéant, on peut inclure dans la solution de décontamination un agent de décontamination supplémentaire, acceptable dans l'environnement oculaire, pour améliorer le spectre des propriétés décontaminantes de la solution de peroxyde d'hydrogène. Ces agents supplémentaires de décontamination sont présents en une quantité qui ne nuit pas aux autres composants. Lorsqu'il est présent, cet agent de décontamination supplémentaire est présent en une quantité à laquelle il est actif pour la décontamination, par exemple 2000 ppm, et mieux entre 10 ppm et 1000 ppm environ.

La présente invention concerne également un procédé d'obtention des solutions stabilisées et tamponnées décrites ci-dessus qui comprend :
1) l'addition à une solution de tampon borate d'une quantité effective d'un agent stabilisant à fonctions phosphoniques choisi parmi les composés phosphoniques sous forme acide, les mélanges de composés phosphoniques sous forme acide et les mélanges d'un ou plusieurs composés phosphoniques sous forme acide avec un ou plusieurs de leurs sels à la condition que la teneur en composés phosphoniques sous forme acide dans le mélange soit au moins égale à 90% en poids par rapport au poids total de l'agent stabilisant à fonctions phosphoniques ;
2) l'addition à la solution obtenue à l'étape 1) d'une solution de peroxyde d'hydrogène ; et
3) le réglage, si nécessaire, du pH de la solution obtenue à l'étape 2) à une valeur comprise entre 5 et 6,5.

Dans une réalisation particulière, le procédé d'obtention d'une solution stabilisée et tamponnée de peroxyde d'hydrogène selon l'invention comprend les étapes suivantes :
1) dissolution d'un tampon de borate et éventuellement d'un agent d'augmentation de la tonicité dans une première fraction de la quantité d'eau totale de la solution finale;
2) addition d'une quantité effective d'un stabilisant à fonctions phosphoniques sous forme acide à la solution obtenue à l'étape 1);
3) addition d'une solution de peroxyde d'hydrogène, contenant éventuellement un stabilisant classique autre que l'agent stabilisant à fonctions phosphoniques, à la solution obtenue à l'étape 2), puis addition d'une seconde fraction de la quantité d'eau totale de la solution finale;
4) réglage, si nécessaire, du pH de la solution obtenue à l'étape 3) à une valeur comprise entre 5 et 6,5; et
5) addition d'une troisième fraction d'eau jusqu'à la quantité voulue pour la solution finale.

Si on utilise un agent filmogène, il est souhaitable de l'ajouter immédiatement à la première fraction d'eau avant ajout du tampon borate et des autres constituants de la solution.

Dans le procédé selon l'invention, il est important de mélanger initialement l'agent stabilisant à fonctions phosphoniques sous forme acide au tampon borate puis d'ajouter ultérieurement le peroxyde d'hydrogène. Il est également important, le cas échéant, de procéder à un ajustage du pH de la solution dans une gamme proche de 6.

### Exemple 1

On prépare une solution aqueuse stabilisée et tamponnée de peroxyde d'hydrogène ayant la composition suivante :

### Composition

- Solution aqueuse concentrée de H₂O₂ 30% 10 g
   massique (stabilisée par : acide phosphorique (85%) 600 mg/kg
   stannate de sodium) 250 mg/kg
- Borax Na₂ B₄O₇, 10H₂O 0,06 g
- Acide borique H₃ BO₃ 0,12 g
- Chlorure de sodium NaCl 0,73 g
- DEQUEST® 2000 (solution à 50% d'acide 0,05 g
   amino tri(méthylène phosphonique) (soit 0,025 g en acide)
- Eau purifiée qsp (déminéralisée) 100 g

La solution de peroxyde est préparée de la façon suivante :
1) on effectue une dissolution des constituants : borax, acide borique et chlorure de sodium dans une fraction correspondant approximativement à 80% de la quantité d'eau totale qui sera utilisée pour compléter à 100 g;
2) on ajoute ensuite le DEQUEST® 2000 (sous forme de solution aqueuse à 50% d'acide);
3) on ajoute ensuite le peroxyde d'hydrogène à 10% de l'eau totale. A ce stade, le pH est de l'ordre de 5 à 5,2;
4) on ajuste le pH à 6 par addition de soude;
5) on complète à 100 g par ajout d'eau (10% restants) Osmolalité finale : 1100.

Comme indiqué ci-dessus, il est important dans le procédé :
- de mélanger le polyacide phosphonique au mélange des sels puis d'ajouter le peroxyde ultérieurement; et
- de procéder à un ajustage du pH de la solution dans une gamme proche de 6.

### Exemples 2 à 4

On prépare de la même manière des solutions aqueuses stabilisées et tamponnées ayant les compositions suivantes :

| | *Exemple 2* | *Exemple 3* | *Exemple 4* |
|---|---|---|---|
| - Solution aqueuse de H₂O₂ 30% massique | 10 g | 10 g | 10 g |
| - Acide borique | 0,12 g | 0,12 g | 0,12 g |
| - Borate de sodium | 0,034 g | 0,034 g | 0,034 g |
| - Chlorure de sodium | 0,73 g | 0,73 g | 0,73 g |
| - DEQUEST® 2000 (solution à 50% d'acide amino tri(méthylène phosphonique) | 0,05 g | 0,05 g | - |
| - DEQUEST® 2016 (solution à 21 % d'acide 1-hydroxy éthylène-1,1-diphosphonique et 30% de sel tétrasodique) | - | 0,004 g | - |
| - DEQUEST® 2060 S (solution à 50% d'acide d'éthylène triamine penta(méthylène phosphonique)) | - | 0,0015 g | - |
| - DEQUEST® 2041 (Solide à 92% en acide éthylène diamine tétra(méthylène phosphonique)) | - | - | 0,05 g |
| - MERQUAT® 2200 | 0,01 g | - | - |
| - H₂O qsp | 100 g | 100 g | 100 g |
| - Ajustage pH (NaOH qs) | 6,30 | 6,30 | 6,30 |

### Exemple comparatifs A à C

On prépare dans les mêmes conditions que dans l'exemple 1 des solutions de peroxyde d'hydrogène ayant les compositions suivantes :

| | *Exemple comparatif A* | *Exemple comparatif B* | *Exemple comparatif C* |
|---|---|---|---|
| - Solution aqueuse de H₂O₂ à 30% massique | 10 g | 10 g | 10 g |
| - Acide borique | - | - | 0,12 g |
| - Borate de sodium | - | - | 0,034 g |
| - Phosphate monosodique | - | 0,062 g | - |
| - Phosphate disodique | - | 0,10 g | - |
| - Chlorure de sodium | 0,73 g | 0,50 g | 0,73 g |
| - DEQUEST® 2000 | 0,05 g | - | - |
| - DEQUEST® 2006 | - | 0,05 g | 0,05 g |
| - MERQUAT® 2200 | - | 0,01 g | 0,01 g |
| - Eau purifiée qsp | 100 g | 100 g | 100 g |
| - pH | | 6,29 | 6,26 |

Le MERQUAT® 2200 est un polymère cationique préparé à partir du chlorure de diméthyl diallyl ammonium et de l'acrylamide. Il joue le rôle d'agent filmogène.

### Essai de stabilité thermique

On a mesuré, dans des conditions comparatives la stabilité thermique des solutions des exemples 1 à 4 et des exemples comparatifs A à C. Les résultats sont donnés dans le tableau I ci-dessous.

**TABLEAU I**

| Perte en peroxyde (%) | | | | | | | |
|---|---|---|---|---|---|---|---|
| | *Ex. 1* | *Ex. 2* | *Ex. 3* | *Ex. 4* | *Ex. A* | *Ex. B* | *Ex. C* |
| 7 jours à 45°C | - | 0,2 | 0,8 | 0 | - | - | - |
| 60°C | 2,2 | 0,44 | 0,8 | 0 | 6,24 | - | - |
| 14 jours à 45°C | - | 1,4 | 1,25 | 0,67 | - | 3,65 | 4,52 |
| 60°C | 4 | 4,6 | 4,68 | 2,53 | 16,2 | 10,3 | 10,55 |
| 28 jours à 45°C | 2 | 3,8 | 4,2 | 1,6 | 3,5 | - | - |

Cet essai de stabilité thermique est réalisé en plaçant 2 flacons (test à 60°C) et 3 flacons (test à 45°C), dont un flacon stérile par lot, de chacune des solutions dans une étuve à la température et pendant la durée indiquées. A la fin de l'essai on dose le peroxyde d'hydrogène avec du permanganate de potassium.

Il ressort des résultats du tableau I que les solutions selon l'invention sont beaucoup plus stables thermiquement que les solutions des exemples comparatifs A à C. En outre le sel DEQUEST® 2006 ne donne aucune synergie avec le tampon borate.

### Tests de neutralisation

On a également effectué des tests de neutralisation des solutions de l'exemple 1 et d'une solution AOSEPT® du commerce lot G 1024 262.

On a neutralisé 50 échantillons de 8 ml de solution de l'exemple 1 et 50 échantillons de 8 ml de la solution AOSEPT® par mise en contact pendant 6 heures avec un catalyseur sous forme de disque de platine du système AOSEPT® (le procédé de fabrication de ce disque est décrit dans le brevet des EU n° 3 912 451).

Le peroxyde d'hydrogène résiduel est déterminé de la façon suivante.

Après 6 heures de neutralisation on prélève 6,44 ml de solution neutralisée de chaque échantillon et on ajoute 0,56 ml de réactif au sultate de titane SII. On centrifuge 20 mn à 3000 t/mn et on lit l'absorbance effectuée à 410 nm par rapport à celle de l'eau.

On réalise une gamme étalon de peroxyde d'hydrogène à 6, 9, 12, 18, 24 et 30 ppm à partir d'une solution à 300 ppm, en en prélèvant 0,2 - 0,3 - 0,4 - 0,6 - 0,8 et 1 ml, et en ajustant à 100 ml avec de l'eau déminéralisée. Dans une fiole de 25 ml, on introduit 2 ml de réactif SII puis on ajuste à 25 ml avec la solution diluée d'H₂O₂. On lit l'absorbance à 410 nm par rapport à celle de l'eau.

On trace la courbe de la concentration de H₂O₂ en fonction de l'absorbance. On obtient une droite A. La teneur en peroxyde d'hydrogène est lue sur la droite.

### Réactif au sulfate de titane SII

SI : à 1 g d'oxyde de titane on ajoute 183 g d'acide sulfurique concentré et on chauffe jusqu'à dissolution complète.

SII : 5 ml de SI sont ensuite dilués dans 25 ml d'eau distillée.

Les résultats sont donnés dans l'annexe 1.

Avec la solution de l'exemple 1, 7 essais sur 50 donnent une solution finale dont la teneur en H₂O₂ résiduel est supérieure à 20 ppm et un seul essai conduit à une teneur supérieure à 25 ppm. Au contraire avec la solution AOSEPT®, 33 essais sur 50 conduisent à une teneur résiduelle en H₂O₂ supérieure à 20 ppm et 23 à une teneur supérieure à 25 ppm.

Il apparaît clairement que les solutions de l'invention permettent d'obtenir une meilleure neutralisation que le système AOSEPT® du commerce.

Après neutralisation le pH de la solution de l'exemple 2 selon l'invention est de 7,2 et l'Osmolalité de 275 alors que dans le cas de la solution AOSEPT® le pH est de 6,7 et l'Osmolalité de 290.

| **Annexe 1** | | |
|---|---|---|
| (Exemple 1) H₂O₂ résiduel en ppm | | Aosept H₂O₂ résiduel en ppm |
| 1 | 7,9 | 9,4 |
| 2 | 4,8 | 12,3 |
| 3 | 5,7 | 7,6 |
| 4 | 7,4 | 19,8 |
| 5 | 18,8 | 20,2 |
| 6 | 16 | 25,7 |
| 7 | 12,7 | 31 |
| 8 | 16,8 | 19 |
| 9 | 9,4 | 26,2 |
| 10 | 12 | 33,1 |
| 11 | 13 | 26,9 |
| 12 | 14,6 | 23,3 |
| 13 | 11,6 | 22,6 |
| 14 | 15,7 | 35,3 |
| 15 | 21,2 | 34,9 |
| 16 | 16,2 | 20,3 |
| 17 | 20,3 | 39,4 |
| 18 | 16,5 | 36,1 |
| 19 | 16,7 | 37,6 |
| 20 | 17,4 | 20,5 |
| 21 | 16 | 40,6 |
| 22 | 21,8 | 46,7 |
| 23 | 22,6 | 25,7 |
| 24 | 17,7 | 36,3 |
| 25 | 18,1 | 26,4 |
| 26 | 12,3 | 22,6 |
| 27 | 11,9 | 31,9 |
| 28 | 17,4 | 22 |
| 29 | 14,4 | 27,1 |
| 30 | 14,4 | 29,5 |
| 31 | 17,4 | 28,5 |
| 32 | 17,3 | 33,2 |
| 33 | 17,5 | 19 |
| 34 | 17,5 | 22,4 |
| 35 | 15,8 | 19,9 |
| 36 | 17 | 16 |
| 37 | 12,4 | 28,9 |
| 38 | 16,3 | 19,8 |
| 39 | 16,8 | 24,9 |
| 40 | 21,6 | 22,1 |
| 41 | 15,2 | 17,5 |
| 42 | 17,3 | 20,6 |
| 43 | 10,7 | 13,9 |
| 44 | 15,6 | 16,8 |
| 45 | 9,6 | 23,7 |
| 46 | 16,8 | 21,6 |
| 47 | 19,6 | 40,5 |
| 48 | 16,6 | 33,4 |
| 49 | 21,6 | 36,7 |
| 50 | 25,1 | 24,5 |

## Revendications

1. Solution aqueuse, stabilisée et tamponnée, de décontamination des lentilles de contact, comprenant 0,5 à 6% en poids, de préférence 2 à 6% et mieux environ 3% en poids de peroxyde d'hydrogène par rapport au poids total de la composition, caractérisée en ce qu'elle comprend en outre :
a) une quantité stabilisante d'un agent stabilisant à fonctions phosphoniques choisi; et
b) une quantité effective d'un tampon borate, ladite solution ayant un pH compris entre 5 et 6,5.

2. Composition selon la revendication 1, caractérisée en ce que l'agent stabilisant à fonctions phosphoniques est introduit sous forme acide lors de la préparation de la solution.

3. Solution selon la revendication 1 ou 2, caractérisée en ce que l'agent stabilisant à fonctions phosphoniques est choisi parmi les acides phosphoniques répondant aux formules : 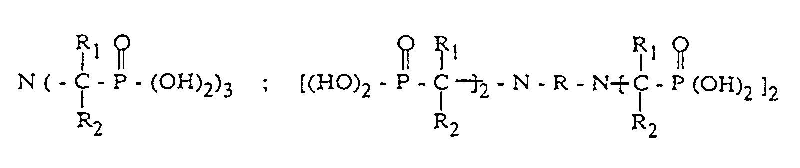 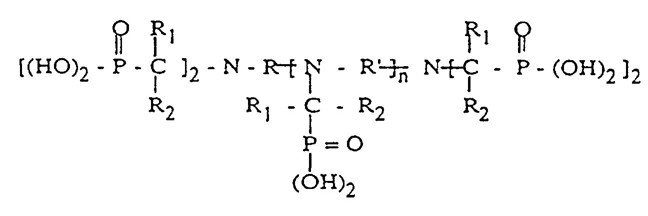 et 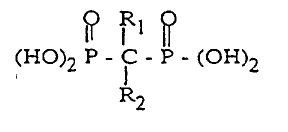 et leurs sels de métaux alcalins, dans lesquelles R₁ et R₂ sont, indépendamment l'un de l'autre, l'hydrogène, un groupe alkyle en C₁-C₄ ou un groupe hydroxyle, R et R' sont, indépendamment l'un de l'autre, un groupe alkylidène en C₁-C₆ et n est un entier de 1 à 4.

4. Solution selon l'une quelconque des revendications 1 à 3 caractérisée en ce qu'elle contient 0,003 à 0,2%, de préférence 0,006 à 0,1% et mieux 0,01 à 0,06% en poids d'agent stabilisant à fonctions phosphoniques par rapport au poids total de la solution.

5. Solution selon l'une quelconque des revendications 1 à 4 caractérisée en ce que le tampon borate est choisi parmi l'acide borique, le borate de sodium, le tétraborate de potassium, le métaborate de potassium, le borax et leurs mélanges.

6. Solution selon l'une quelconque des revendications 1 à 5 caractérisée par le fait qu'elle contient 0,05 à 2,5% en poids et de préférence 0,1 à 1,5% en poids de tampon borate par rapport au poids total de la solution.

7. Solution selon l'une quelconque des revendications précédentes caractérisée en ce qu'elle a un pH compris entre 5,5 et 6,5, et mieux entre 5,8 et 6,2.

8. Solution selon la revendication 7 caractérisée en ce qu'elle a un pH d'environ 6.

9. Solution selon l'une quelconque des revendications précédentes caractérisée en ce qu'elle comprend un ou plusieurs agents stabilisants différents de l'agent stabilisant à fonctions phosphoniques.

10. Solution selon l'une quelconque des revendications précédentes caractérisée en ce qu'elle comporte en outre un agent filmogène.

11. Solution selon l'une quelconque des revendications 1 à 10 caractérisée en ce que l'agent stabilisant à fonctions phosphoniques est uniquement sous forme acide.

12. Procédé d'obtention d'une solution stabilisée et tamponnée de peroxyde d'hydrogène ayant un pH compris entre 5 et 6,5 caractérisé en ce qu'il comprend :
1) l'addition à une solution de tampon borate d'une quantité effective d'un agent de stabilisation à fonctions phosphoniques choisi parmi les composés phosphoniques sous forme acide, les mélanges de composés phosphoniques sous forme acide et les mélanges d'un ou plusieurs composés phosphoniques sous forme acide avec un ou plusieurs de leurs sels à la condition que la teneur en composés phosphoniques sous forme acide dans le mélange soit au moins égale à 90% en poids par rapport au poids total de l'agent stabilisant à fonctions phosphoniques;
2) l'addition à la solution obtenue à l'étape 1) d'une solution de peroxyde d'hydrogène; et
3) le réglage, si nécessaire, du pH de la solution obtenue à l'étape 2) à une valeur comprise entre 5 et 6,5.

13. Procédé d'obtention d'une solution stabilisée et tamponnée de peroxyde d'hydrogène ayant un pH compris entre 5 et 6,5 caractérisé en ce qu'il comprend les étapes suivantes :
1) dissolution du tampon de borate et éventuellement d'un agent d'augmentation de la tonicité dans une première fraction de la quantité d'eau totale de la solution finale;
2) addition d'une quantité effetive d'un agent de stabilisation à fonctions phosphoniques à la solution obtenue à l'étape (1), ledit agent étant choisi parmi les composés phosphoniques sous forme acide, les mélanges de composés phosphoniques sous forme acide et les mélanges d'un ou plusieurs composés phosphoniques sous forme acide avec un ou plusieurs de leurs sels à la condition que la teneur en composés phosphoniques sous forme acide dans le mélange soit au moins égale à 90% en poids par rapport au poids total de l'agent stabilisant à fonctions phosphoniques ;
3) addition du peroxyde d'hydrogène, contenant éventuellement un stabilisant classique autre que l'agent stabilisant à fonctions phosphoniques à la solution obtenue à l'étape 2), puis addition d'une seconde fraction de la quantité d'eau totale de la solution finale ;
4) réglage, si nécessaire, du pH de la solution obtenue à l'étape 3) à une valeur comprise entre 5 et 6,5 ; et
5) addition d'une troisième fraction d'eau jusqu'à obtention de la quantité voulue pour la solution finale.

14. Procédé selon la revendication 12 ou 13, caractérisé en ce que la solution a un pH compris entre 5,5 et 6,5 de préférence 5,8 à 6,2 et l'étape éventuelle de réglage du pH de la solution consiste à régler le pH à une valeur comprise entre 5,5 et 6,5 et de préférence 5,8 à 6,2.

15. Utilisation d'une solution selon l'une quelconque des revendications 1 à il pour la décontamination de lentilles de contact.

## Claims

1. Stabilised and buffered aqueous solution for decontaminating contact lenses, comprising 0.5 to 6% by weight, preferably 2 to 6% and more preferably about 3% by weight of hydrogen peroxide based on the total weight of the composition, characterised in that it also comprises :
(a) a stabilising amount of a stabilising agent with phosphonic functions, selected from phosphonic compounds in acid form, mixtures of phosphonic compounds in acid form and mixtures of one or more phosphonic compounds in acid form with one or more of their salts on condition that the concentration of phosphonic compounds in acid form in the mixture is at least 90% by weight based on the total weight of stabilising agent with phosphonic functions; and
(b) an effective amount of a borate buffer, said solution having a pH between 5 and 6.5.

2. Composition according to claim 1, characterised in that the stabilising agent with phosphonic functions is introduced in acid form during the preparation of the solution.

3. Solution according to claim 1 or 2, characterised in that the stabilising agent with phosphonic functions is selected from the phosphonic acids with the following formulae : 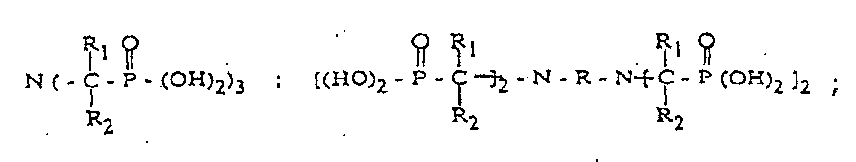 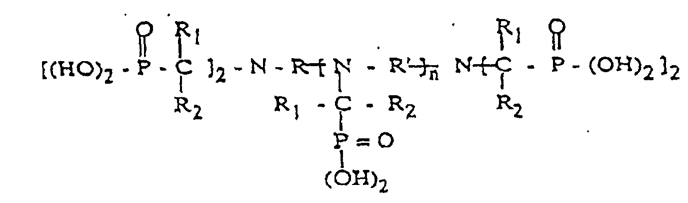 and 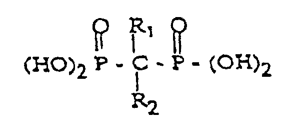 and their alkali metal salts, in which R₁ and R₂ are independently hydrogen, a C₁-C₄ alkyl group or a hydroxyl group, R and R' are independently a C₁-C₆ alkylidene group and n is an integer from 1 to 4.

4. Solution according to any one of claims 1 to 3, characterised in that it comprises 0.003 to 0.2%, preferably 0.006 to 0.1% and more preferably 0.01 to 0.06% by weight of stabilising agent with phosphonic functions based on to the total weight of the solution.

5. Solution according to any one of claims 1 to 4, characterised in that the borate buffer is selected from boric acid, sodium borate, potassium tetraborate, potassium metaborate, borax and their mixtures.

6. Solution according to any one of claims 1 to 5, characterised in that it comprises 0.05 to 2.5% by weight and preferably 0.1 to 1.5% by weight of borate buffer based on to the total weight of the solution.

7. Solution according to any one of the preceding claims, characterised in that it has a pH between 5.5 and 6.5, preferably between 5.8 and 6.2.

8. Solution according to claim 7, characterised in that it has a pH of about 6.

9. Solution according to any one of the preceding claims, characterised in that it comprises one or more stabilising agents different from the stabilising agent with phosphonic functions.

10. Solution according to any one of the preceding claims, characterised in that it also comprises a film like agent.

11. Solution according to any one of claims 1 to 10, characterised in that the stabilising agent with phosphonic functions is only present in acid form.

12. Method for obtaining a stabilised and buffered hydrogen peroxide solution with a pH between 5 and 6.5, characterised in that it comprises :
1) the addition to a borate buffer solution of an effective amount of a stabilising agent with phosphonic functions selected from phosphonic compounds in acid form, mixtures of phosphonic compounds in acid form and mixtures of one or more phosphonic compounds in acid form with one or more of their salts on condition that the concentration of phosphonic compounds in acid form in the mixture is at least 90% by weight based on the total weight of stabilising agent with phosphonic functions;
2) the addition to the solution obtained in step 1) of a hydrogen peroxide solution; and
3) the adjustment, if required, of the pH of the solution obtained in step 2) to a value between 5 and 6.5.

13. Method for obtaining a stabilised and buffered hydrogen peroxide solution with a pH between 5 and 6.5, characterised in that it comprises the following steps :
1) dissolution of the borate buffer and optionally an agent for increasing the stimulation into a first fraction of the total amount of water of the final solution;
2) addition of an effective amount of a stabilising agent with phosphonic functions to the solution obtained in step 1), said agent being selected from phosphonic compounds in acid form, mixtures of phosphonic compounds in acid form and mixtures of one or more phosphonic compounds in acid form with one or more of their salts on condition that the concentration of phosphonic compounds in acid form in the mixture is at least 90% by weight based on to the total weight of stabilising agent with phosphonic functions;
3) addition of hydrogen peroxide, optionally comprising a conventional stabiliser other than the stabilising agent with phosphonic functions, to the solution obtained in step 2), then addition of a second fraction of the total amount of water of the final solution;
4) adjustment, if required, of the pH of the solution obtained in step 3) to a value between 5 and 6.5; and
5) addition of a third fraction of water until the desired amount of the final solution is obtained.

14. Method according to claim 12 or 13, characterised in that the solution has a pH between 5.5 and 6.5, preferably between 5.8 and 6.2, and the optional step of adjusting the pH of the solution consists of adjusting the pH to a value between 5.5 and 6.5 and preferably between 5.8 and 6.2.

15. Use of a solution according to any one of claims 1 to 11 for decontaminating contact lenses.

## Patentansprüche

1. Stabilisierte und gepufferte wässrige Lösung zur Dekontaminierung von Kontaktlinsen, enthaltend 0,5 bis 6 Gew.-%, vorzugsweise 2 bis 6 und noch mehr bevorzugt 3 Gew.-% Wasserstoffperoxid, bezogen auf das Gesamtgewicht der Zusammensetzung, dadurch gekennzeichnet, dass sie außerdem enthält:
a) eine stabilisierende Menge eines Stabilisierungsmittels mit Phosphonfunktionen, ausgewählt aus den Phosphonverbindungen in Säureform, den Mischungen von Phosphonverbindungen in Säureform und den Mischungen einer oder mehrerer Phosphonverbindungen in Säureform mit einem oder mehreren Salzen davon mit der Maßgabe, dass der Gehalt an Phosphonverbindungen in Säureform in der Mischung mindestens gleich 90 Gew.-% beträgt, bezogen auf das Gesamtgewicht des Stabilisierungsmittels mit Phosphonfunktionen, und
b) eine wirksame Menge eines Boratpuffers, wobei die Lösung einen pH-Wert zwischen 5 und 6,5 aufweist.

2. Zusammensetzung nach Anspruch 1, dadurch gekennzeichnet, dass das Stabilisierungsmittel mit Phosphonfunktionen bei der Herstellung der Lösung in Säureform eingeführt wird.

3. Lösung nach Anspruch 1 oder 2, dadurch gekennzeichnet, dass das Stabilisierungsmittel mit Phosphonfunktionen aus den Phosphonsäuren mit den Formeln: 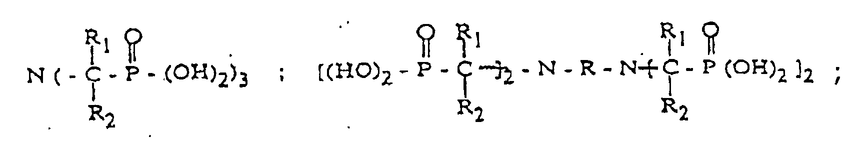 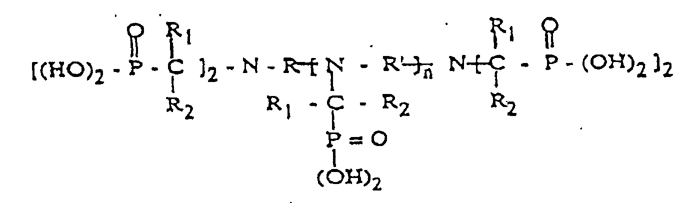 und 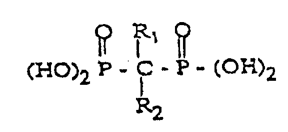 und deren Alkalimetallsalzen ausgewählt ist, wobei R₁ und R₂ unabhängig voneinander Wasserstoff, eine C₁-C₄-Alkylgruppe oder eine Hydroxylgruppe sind, R und R' unabhängig voneinander eine C₁-C₆-Alkylidengruppe sind und n eine ganze Zahl von 1 bis 4 ist.

4. Lösung nach einem der Ansprüche 1 bis 3, dadurch gekennzeichnet, dass sie 0,003 bis 0,2, vorzugsweise 0,006 bis 0,1 und noch mehr bevorzugt 0,01 bis 0,06 Gew.-% eines Stabilisierungsmittels mit Phosphonfunktionen, bezogen auf das Gesamtgewicht der Lösung, enthält.

5. Lösung nach einem der Ansprüche 1 bis 4, dadurch gekennzeichnet, dass der Boratpuffer aus Borsäure, Natriumborat, Kaliumtetraborat, Kaliummetaborat, Borax und Mischungen davon ausgewählt ist.

6. Lösung nach einem der Ansprüche 1 bis 5, dadurch gekennzeichnet, dass sie 0,05 bis 2,5 Gew.-% und vorzugsweise 0,1 bis 1,5 Gew.-% Boratpuffer, bezogen auf das Gesamtgewicht der Lösung, enthält.

7. Lösung nach einem der vorhergehenden Ansprüche, dadurch gekennzeichnet, dass sie einen pH-Wert zwischen 5,5 und 6,5 und noch mehr bevorzugt zwischen 5,8 und 6,2 aufweist.

8. Lösung nach Anspruch 7, dadurch gekennzeichnet, dass sie einen pH-Wert von etwa 6 aufweist.

9. Lösung nach einem der vorhergehenden Ansprüche, dadurch gekennzeichnet, dass sie ein oder mehrere Stabilisierungsmittel enthält, die vom Stabilisierungsmittel mit Phosphonfunktionen verschieden sind.

10. Lösung nach einem der vorhergehenden Ansprüche, dadurch gekennzeichnet, dass sie außerdem einen Filmbildner enthält.

11. Lösung nach einem der Ansprüche 1 bis 10, dadurch gekennzeichnet, dass das Stabilisierungsmittel mit Phosphonfunktionen nur in Säureform vorliegt.

12. Verfahren zur Herstellung einer stabilisierten und gepufferten Wasserstoffperoxid-Lösung mit einem pH-Wert zwischen 5 und 6,5, dadurch gekennzeichnet, dass sie umfasst:
1) die Zugabe einer wirksamen Menge eines Stabilisierungsmittels mit Phosphonfunktionen, ausgewählt aus den Phosphonverbindungen in Säureform, den Mischungen von Phosphonverbindungen in Säureform und den Mischungen einer oder mehrerer Phosphonverbindungen in Säureform mit einem oder mehreren Salzen davon mit der Maßgabe, dass der Gehalt an Phosphonverbindungen in Säureform in der Mischung mindestens gleich 90 Gew.-% beträgt, bezogen auf das Gesamtgewicht des Stabilisierungsmittels mit Phosphonfunktionen, zu einer Boratpufferlösung,
2) die Zugabe einer Wasserstoffperoxid-Lösung zu der in Schritt 1) erhaltenen Lösung und
3) die Regelung, falls erforderlich, des pH-Werts der in Schritt 2) erhaltenen Lösung auf einen Wert zwischen 5 und 6,5.

13. Verfahren zur Herstellung einer stabilisierten und gepufferten Wasserstoffperoxid-Lösung mit einem pH-Wert zwischen 5 und 6,5, dadurch gekennzeichnet, dass es die folgenden Schritte umfasst:
1) das Lösen des Boratpuffers und gegebenenfalls eines Mittels zur Erhöhung der Tonizität in einem ersten Anteil der gesamten Wassermenge der fertigen Lösung,
2) die Zugabe einer wirksamen Menge eines Stabilisierungsmittels mit Phosphonfunktionen zu der in Schritt (1) erhaltenen Lösung, wobei das Mittel ausgewählt ist aus den Phosphonverbindungen in Säureform, den Mischungen von Phosphonverbindungen in Säureform und den Mischungen einer oder mehrerer Phosphonverbindungen in Säureform mit einem oder mehreren Salzen davon mit der Maßgabe, dass der Gehalt an Phosphonverbindungen in Säureform in der Mischung mindestens gleich 90 Gew.-% beträgt, bezogen auf das Gesamtgewicht des Stabilisierungsmittels mit Phosphonfunktionen,
3) die Zugabe von Wasserstoffperoxid, das gegebenenfalls ein klassisches Stabilisierungsmittel enthält, das von dem Stabilisierungsmittel mit Phosphonfunktionen der in Schritt 2) erhaltenen Lösung verschieden ist, anschließend die Zugabe eines zweiten Anteils der gesamten Wassermenge der fertigen Lösung,
4) die Regelung, falls erforderlich, des pH-Werts der in Schritt 3) erhaltenen Lösung auf einen Wert zwischen 5 und 6,5, und
5) die Zugabe eines dritten Anteils Wasser bis zum Erhalt des gewünschten Volumens der fertigen Lösung.

14. Verfahren nach Anspruch 12 oder 13, dadurch gekennzeichnet, dass die Lösung einen pH-Wert zwischen 5,5 und 6,5, vorzugsweise zwischen 5,8 und 6,2 aufweist und der Schritt der eventuellen Regelung des pH-Werts der Lösung aus der Regelung des pH-Werts auf einen Wert zwischen 5,5 und 6,5 und vorzugsweise zwischen 5,8 und 6,2 besteht.

15. Verwendung einer Lösung nach einem der Ansprüche 1 bis 11 zur Dekontaminierung von Kontaktlinsen.
